# EUROPÄISCHE PATENTSCHRIFT

(11) **EP 0 666 252 B1**
(45) Veröffentlichungstag und Bekanntmachung des Hinweises auf die Patenterteilung: **02.10.2002**
(21) Anmeldenummer: 95100675.8
(22) Anmeldetag: 19.01.1995
(51) Int. Cl.: C07C 279/22, A61K 31/155, G01N 33/50

(54) **Perfluoralkylgruppen tragende phenyl-substituierte Alkylcarbonsäure-guanidide, Vefahren zu ihrer Herstellung, ihre Verwendung als Medikament oder Diagnostikum sowie sie enthaltendes Medikament**
Phenyl substituted alkyl carboxylic acid guanidines with perfluoroalkyl groups, process for their preparation, their use as medicament or diagnostic agent as well as medicament containing the same
Alkyl carboxylique acide guanidines avec des groupes perfluoro-alkyls substituées par des groupes phényles, procédé de leur préparation, leur utilisation comme médicament ou agent diagnostique ainsi que médicament les contenant

(30) Priorität: 25.01.1994 DE 4402057; 19.04.1994 DE 4413615
(43) Veröffentlichungstag der Anmeldung: 09.08.1995
(73) Patentinhaber: HOECHST AKTIENGESELLSCHAFT, 65929 Frankfurt am Main (DE)
(72) Erfinder: Schwark, Jan-Robert, Dr., D-65929 Frankfurt (DE); Kleemann, Heinz-Werner, Dr., D-65474 Bischofsheim (DE); Lang, Hans-Jochen, Dr., D-65719 Hofheim (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 087 218
- EP-A- 0 556 672
- EP-A- 0 627 413
- WO-A-84/00875
- GB-A- 2 055 093

## Beschreibung

Die Erfindung betrifft Perfluoralkylgruppen tragende phenylsubstituierte Alkylcarbonsäure-guanidide der Formel I 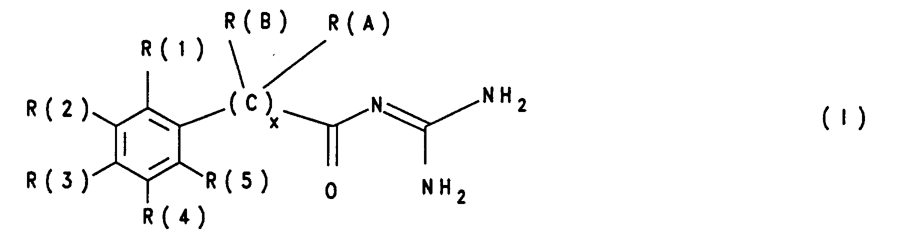 worin bedeuten:
R(A) Wasserstoff, F, Cl, Br, I, CN, OR(6), (C₁-C₄)-Alkyl, -C_{b}F_{2b+1} oder NR(7)R(8),
   b 1, 2, 3 oder 4;
   R(6) Wasserstoff, (C₁-C₄)-Alkyl, -C_{b}F_{2b+1}, Phenyl oder Benzyl, wobei die Aromaten nicht substituiert oder substituiert sind mit 1 - 3 Substituenten ausgewählt aus der Gruppe bestehend aus F, Cl, CF₃, Methyl, Methoxy und NR(9)R(10);
   R(9) und R(10)
   H, CH₃ oder CF₃;
   R(7) und R(8) unabhängig voneinander wie R(6) definiert,
R(B) unabhängig wie R(A) definiert,
X 2,
R(1) Wasserstoff, (C₁-C₄)-Alkyl, -(O)ₜ-C_{b}F_{2b+1}, F oder Cl;
   b 1, 2, 3 oder 4;
   t Null oder 1;
R(2), R(3), R(4), R(5)
   unabhängig voneinander wie R(1) definiert.
jedoch unter der Bedingung,
daß mindestens einer der Substituenten R(1), R(2), R(3), R(4) und R(5) eine -C_{b}F_{2b+1}-Gruppe ist,
sowie deren pharmazeutisch verträgliche Salze.

Enthält die Verbindung der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkyl- und Perfluoralkyl-reste können sowohl geradkettig wie verzweigt vorliegen.

Die Erfindung betrifft weiterhin ein Verfahren zur Herstellung der Verbindung I, dadurch gekennzeichnet, daß man eine Verbindung der Formel II 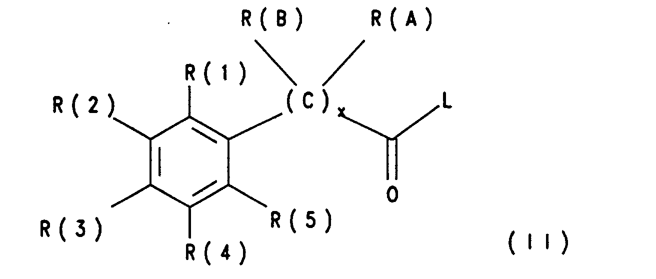 mit Guanidin umsetzt, worin R(1) bis R(5) und R(A) sowie R(B) die in Anspruch 1 angegebene Bedeutung besitzen und L für eine leicht nucleophil substituierbare Fluchtgruppe steht,
und daß man gegebenenfalls in ein pharmazeutisch verträgliches Salz überführt.

Die aktivierten Säurederivate der Formel II, worin L eine Alkoxy-, vorzugsweise eine Methoxygruppe, eine Phenoxygruppe, Phenylthio, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Carbonsäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 bis 367 (1962)], die gemischten Anhydride II mit Cl-COOC₂H₅ oder Tosylchlorid in Gegenwart von Triethylamin in einem inerten Lösungsmittel, wie auch die Aktivierungen von Carbonsäuren mit Dicyclohexylcarbodiimid (DCC) oder mit O-[(Cyano(ethoxycarbonyl)methylen)amino]-1,1,3,3-tetramethyluronium-tetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Carbonsäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z.B. in Form von überschüssigen Guanidin zur Abbindung der Halogenwasserstoffsäure.

Ein Teil der zugrundeliegenden Carbonsäurederivate der Formel II ist bekannt und in der Literatur beschrieben. Die unbekannten Verbindungen der Formel II können nach literaturbekannten Methoden hergestellt werden. Die erhaltenen Carbonsäuren werden nach einer der oben beschriebenen Verfahrensvarianten zu erfindungsgemäßen Verbindungen I umgesetzt.

Die Einführung einiger Substituenten gelingt durch literaturbekannte Methoden des Palladium-vermittelten cross-couplings von Arylhalogeniden bzw. Aryltriflaten mit beispielsweise Organostannanen, Organoboronsäuren, Organoboranen, Organokupfer- bzw. -zink-verbindungen oder terminalen Alkinen.

Acylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 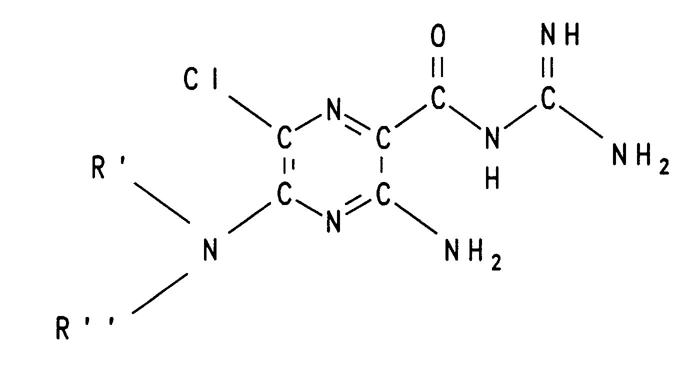 Amilorid: R',R'' = H
Dimethylamilorid: R',R'' = CH₃
Ethylisopropylamilorid: R' = C₂H₅, R'' = CH(CH₃)₂

Darüber hinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988) (book of abstracts)]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288), der EP-Offenlegungsschrift 556 674 (HOE 92/F 034) und der US-Patentschrift 3 780 027 sind Benzoylguanidine beschrieben. Die erfindungsgemäßen Verbindungen unterscheiden sich von den daraus bekannten durch die -CR(A)R(B)-Gruppe zwischen CO-Gruppe und Phenylrest.

Aus der WO 84/00875 sind unter anderem ähnliche Acylguanidine bekannt, die jedoch nur in Aufzählungen von ebenfalls geeigneten Verbindungen ohne physikalische Daten auch Perfluoralkylgruppen erwähnen. Außerdem ist in keinem der Ausführungsbeipiele eine Perfluoralkylgruppe enthalten. Ferner sind keine Propionsäureguanidide bekannt oder nahegelegt.

Da sich bisher alle antiarrhythmisch wirkenden Guanidine von Aryl- bzw. Heteroarylcarbonsäuren ableiten, war es daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des Zentralnervensystems, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.
Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise
0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

| Liste der Abkürzungen: | |
|---|---|
| MeOH | Methanol |
| DMF | N,N-Dimethylformamid |
| El | electron impact |
| DCI | Desorption-Chemical Ionisation |
| RT | Raumtemperatur |
| EE | Ethylacetat (EtOAc) |
| mp | Schmelzpunkt |
| HEP | n-Heptan |
| DME | Dimethoxyethan |
| ES | Elektronenspray |
| FAB | Fast Atom Bombardment |
| CH₂Cl₂ | Dichlormethan |
| THF | Tetrahydrofuran |
| eq. | Äquivalent |
| Pd/C | Palladium auf Kohle |
| Pt/C | Platin auf Kohle |
| LDA | Lithiumdiisopropylamin |

### Experimenteller Teil

Allgemeine Vorschrift zur Herstellung von Acyl-guanidinen (I) Variante A: aus Carbonsäuren (II, L = OH)

1.0 eq. des Carbonsäurederivates der Formel II löst bzw. suspendiert man in wasserfreiem THF (5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck im Rotationsverdampfer ab, versetzt mit Wasser, stellt mit 2 N HCI auf pH 6 bis 7 und filtriert das entsprechende Acyl-guanidin (Formel I) ab. Die so erhaltenen Acyl-guanidine können durch Behandeln mit wäßriger, methanolischer oder etherischer Salzsäure oder anderen pharmakologisch verträglichen Säuren in die entsprechenden Salze übergeführt werden.

Allgemeine Vorschrift zur Herstellung von Acyl-guanidinen (I)
Variante B: aus Carbonsäure-alkylestern (II, L = O-Alkyl)

1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (im Rotationsverdampfer) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1: 3-(3-Trifluormethylphenyl)-propionsäure-guanidid

Hydrierung der m-Trifluormethyl-zimtsäure über 10% Pd/C in EE bei RT unter Normaldruck ergab 3-(3-Trifluormethylphenyl)-propionsäure.

1.0 eq. dieser gesättigten Carbonsäure wurde gemäß Variante A mit 1.1 eq. Carbonyldiimidazol und 5 eq. Guanidin umgesetzt.
MS (ES): 260 (M+1)

### Beispiel 2: 3-(2-Trifluormethylphenyl)-propionsäure-guanidid

Hydrierung der o-Trifluormethyl-zimtsäure über 10% Pd/C in EE bei RT unter Normaldruck ergab 3-(2-Trifluormethylphenyl)-propionsäure.

1.0 eq. dieser gesättigten Carbonsäure wurde gemäß Variante A mit 1.1 eq. Carbonyldiimidazol und 5 eq. Guanidin umgesetzt.
MS (ES): 260 (M+1)
mp : 73-80°C

### Beispiel 3: 3-(3-Trifluormethoxyphenyl)-propionsäure-guanidid hydrochlorid

Hydrierung der m-Trifluormethoxyzimtsäure über 10% Pd/C in EtOH bei RT unter Normaldruck ergab 3-(3-Trifluormethoxyphenyl)-propionsäure.

1.0 eq. dieser gesättigten Carbonsäure wurde gemäß Variante A mit 1.1 eq. Carbonyldiimidazol und 5 eq. Guanidin umgesetzt und als Hydrochlorid isoliert.
MS (ES): 276 (M+1)

### Beispiel 4: 3-(4-Trifluormethylphenyl)-propionsäure-guanidid

Hydrierung der p-Trifluormethyl-zimtsäure über 10% Pd/C in EE bei RT unter Normaldruck ergab 3-(4-Trifluormethylphenyl)-propionsäure.

1.0 eq. dieser gesättigten Carbonsäure wurde gemäß Variante A mit 1.1 eq. Carbonyldiimidazol und 5 eq. Guanidin umgesetzt.
MS (ES): 260 (M+1)
mp: 146 - 153°C

### Beispiel 5: 2-Methyl-3-(3-trifluormethylphenyl)propionsäureguanidid hydrochlorid.

### 5 a)

2-Methylmalonsäurediethylester wurde in THF mit 1 eq. Natriumhydrid ins Anion überführt und mit 1.05 eq. 3-Trifluormethylbenzylbromid alkyliert. Nach Standardaufarbeitung erhielt man 2-Methyl-2-(3-trifuormethylbenzyl)malonsäurediethylester.
Farbloses Öl, MS (ES): 333 (M+1)

### 5 b)

Der Diester aus 4 a) wurde in Eisessig/5 N HCI unter Rückfluß verseift und zur 2-Methyl-3-(3-trifluormethylphenyl)propionsäure decarboxyliert.
Farbloses Öl, MS (ES): 233 (M+1)

### 5 c)

Die Carbonsäure aus 4 b) wurde gemäß Variante A ins Guanidid überführt und als Hydrochlorid isoliert.
Amorpher Feststoff, MS (ES): 274 (M+1)

### Beispiel 6: 2-Ethyl-3-(3-trifluormethylphenyl)propionsäureguanidid

### 6 a)

1 eq. 3-(3-Trifluormethylphenyl)propionsäure wurde in THF bei -78°C mit 2 eq. LDA ins Dianion überführt (30 min. rühren bei -78°C, dann 30 min. bei RT). Anschließend gab man bei -78°C 2 eq. Ethyliodid hinzu und rührte bei RT nach. Saure Aufarbeitung mit 2 N HCI und chromatographische Säuberung ergab 2-Ethyl-3-(3-trifluormethylphenyl)propionsäure.

### 6 b)

Die Carbonsäure aus 6 a) wurde gemäß Variante A ins Guanidid überführt und als freie Base isoliert.
Farbloses Öl, MS (ES): 288 (M+1)

### Beispiel 7: 2-lsopropyl-3-(3-trifluormethylphenyl)propionsäureguanidid

Wurde in Analogie zu Beispiel 6 dargestellt.
Farbloses Öl, MS (ES): 302 (M+1)

### Beispiel 8: 2-Isobutyl-3-(3-trifluormethylphenyl)propionsäureguanidid

Wurde in Analogie zu Beispiel 6 dargestellt.
Amorpher Feststoff, MS (ES): 316 (M+1)

### Beispiel 9: 2-Ethyl-3-[3,5-Bis(trifluormethyl)phenyl]propionsäureguanidid

Wurde in Analogie zu Beispiel 5 dargestellt.
Farbloses Öl, MS (ES): 356 (M+1)

### Pharmakologische Daten:

### Inhibition des Na⁺/H⁺-Exchangers von Kaninchenerythozyten:

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrozyten via Na⁺/H⁺-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des Na⁺-Ausgangsgehalts der Erythrozyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCI, 3 KCI, 150 Sucrose, 0,1 Ouabain, 20 Tris-hydroxymethyl-aminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrozyten nach Inkubation errechnet. Der Amiloridhemmbare Natrium-Influx ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

Ergebnisse zur Inhibition des Na⁺/H⁺-Exchangers:

| Beispiel | IC₅₀ ( µmol) |
|---|---|
| 1 | 0.6 |
| 2 | 0.5 |
| 3 | 0.7 |
| 5 | 0.05 |

## Patentansprüche

1. Perfluoralkylgruppen tragende phenylsubstituierte Alkylcarbonsäure-guanidide der Formel I 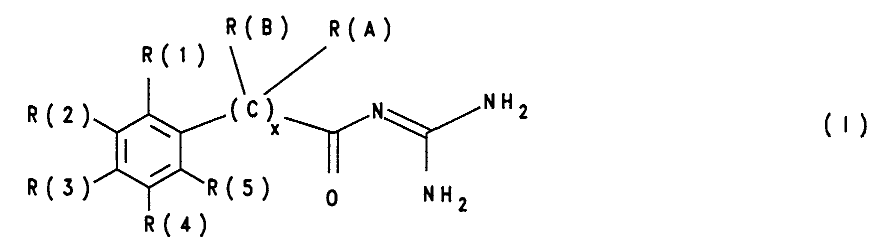 worin bedeuten:
R(A) Wasserstoff, F, Cl, Br, I, CN, OR(6), (C₁-C₄)-Alkyl, -C_{b}F_{2b+1} oder NR(7)R(8),
b 1, 2, 3 oder 4;
R(6) Wasserstoff, (C₁-C₄)-Alkyl, -C_{b}F_{2b+1}, Phenyl oder Benzyl, wobei die Aromaten nicht substituiert oder substituiert sind mit 1 - 3 Substituenten ausgewählt aus der Gruppe bestehend aus F, Cl, CF₃, Methyl, Methoxy und NR(9)R(10);
R(9) und R(10)
H, CH₃ oder CF₃;
R(7) und R(8) unabhängig voneinander wie R(6) definiert,
R(B) unabhängig wie R(A) definiert,
X 2,
R(1) Wasserstoff, (C₁-C₄)-Alkyl, -(O)ₜ-C_{b}F_{2b+1}, F oder Cl;
b 1, 2, 3 oder 4;
t Null oder 1;
R(2), R(3), R(4), R(5) unabhängig voneinander wie R(1) definiert.
jedoch unter der Bedingung,
daß mindestens einer der Substituenten R(1), R(2), R(3), R(4) und R(5) eine
-C_{b}F_{2b+1}-Gruppe ist, sowie deren pharmazeutisch verträgliche Salze.

2. Verfahren zur Herstellung einer Verbindung I nach Anspruch 1, **dadurch gekennzeichnet, daß** man eine Verbindung der Formel II 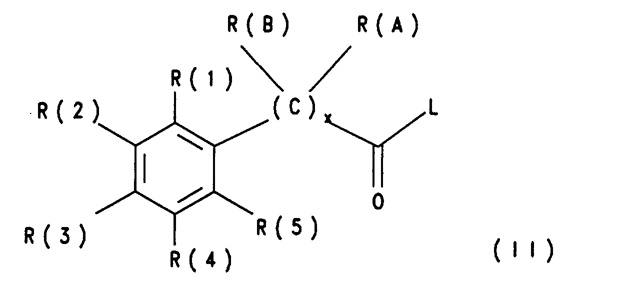 mit Guanidin umsetzt, worin R(1) bis R(5) und R(A) sowie R(B) die in Anspruch 1 angegebene Bedeutung besitzen und L für eine leicht nucleophil substituierbare Fluchtgruppe steht,
und daß man gegebenenfalls in ein pharmazeutisch verträgliches Salz überführt.

3. Verwendung einer Verbindung I nach Anspruch 1 zur Herstellung eines Medikaments zu Behandlung von Arrhythmien.

4. Verwendung einer Verbindung I nach Anspruch 1 zur Herstellung eines Medikaments zur Behandlung oder Prophylaxe des Herzinfarkts.

5. Verwendung einer Verbindung I nach Anspruch 1 zur Herstellung eines Medikaments zur Behandlung oder Prophylaxe der Angina Pectoris.

6. Verwendung einer Verbindung I nach Anspruch 1 zur Herstellung eines Medikaments zur Behandlung oder Prophylaxe von ischämischen Zuständen des Herzens.

7. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

8. Verwendung einer Verbindung I nach Anspruch 1 zur Herstellung eines Medikaments zur Behandlung oder Prophylaxe von ischämischen Zuständen peripherer Organe und Gliedmaßen.

9. Verwendung einer Verbindung I nach Anspruch 1 zur Herstellung eines Medikaments zur Behandlung von Schockzuständen.

10. Verwendung einer Verbindung I nach Anspruch 1 zur Herstellung eines Medikaments zum Einsatz bei chirurgischen Operationen und Organtransplantationen.

11. Verwendung einer Verbindung I nach Anspruch 1 zur Herstellung eines Medikaments zur Konservierung und Lagerung von Transplantaten für chirurgische Maßnahmen.

12. Verwendung einer Verbindung I nach Anspruch 1 zur Herstellung eines Medikaments zur Behandlung von Krankheiten, bei denen die Zellproliferation eine primäre oder sekundäre Ursache darstellt.

13. Heilmittel, enthaltend eine wirksame Menge einer Verbindung I nach Anspruch 1.

## Claims

1. A phenyl-substituted alkylcarboxylic acid guanidide bearing perfluoroalkyl groups, of the formula I 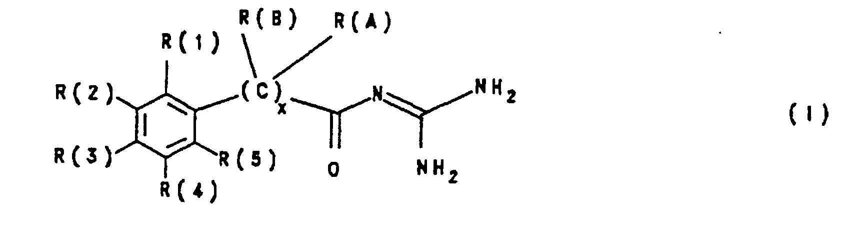 in which:
R(A)is hydrogen, F, Cl, Br, I, CN, OR(6), (C₁-C₄)-alkyl, -C_{b}F_{2b+1}, or NR(7)R(8),
b is 1, 2, 3 or 4;
R(6) is hydrogen, (C₁-C₄)-alkyl, -C_{b}F_{2b+1}, phenyl or benzyl,
where the aromatics are unsubstituted or substituted by 1 - 3 substituents from the group consisting of F, Cl, CF₃, methyl, methoxy and NR(9)R(10);
R(9) and R(10) are
H, CH₃ or CF₃;
R(7) and R(8) independently of one another are defined as R(6);
R(B) independently is defined as R(A),
X is 2,
R(1) is hydrogen, (C₁-C₄)-alkyl, -(O)ₜ-C_{b}F_{2b+1}, F or Cl;
b is 1, 2, 3 or 4;
t is zero or 1;
R(2), R(3), R(4) and R(5) independently of one another are defined as R(1), but with the condition
that at least one of the substituents R(1), R(2), R(3), R(4) and R(5) is a -C_{b}F_{2b+1} group,
as well as pharmaceutically tolerated salts thereof.

2. A process for preparing a compound I as claimed in claim 1, which comprises reacting a compound of the formula II 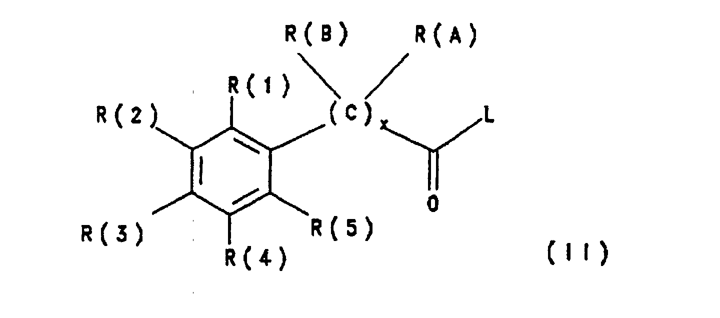 in which R(1) to R(5) and R(A) and also R(B) have the meaning given in claim 1 and L is a leaving group which can readily be substituted nucleophilically, with guanidine,
and which comprises converting said compound into a pharmaceutically tolerated salt, if desired.

3. Use of a compound I as claimed in claim 1 for preparing a medicament for treating arrhythmias.

4. Use of a compound I as claimed in claim 1 for preparing a medicament for the treatment or prophylaxis of cardiac infarction.

5. Use of a compound I as claimed in claim 1 for preparing a medicament for the treatment or prophylaxis of angina pectoris.

6. Use of a compound I as claimed in claim 1 for preparing a medicament for the treatment or prophylaxis of ischemic conditions of the heart.

7. Use of a compound I as claimed in claim 1 for preparing a medicament for the treatment or prophylaxis of ischemic conditions of the peripheral and central nervous system and of stroke.

8. Use of a compound I as claimed in claim 1 for preparing a medicament for the treatment or prophylaxis of ischemic conditions of peripheral organs and limbs.

9. Use of a compound I as claimed in claim 1 for preparing a medicament for the treatment of conditions of shock.

10. Use of a compound I as claimed in claim 1 for preparing a medicament for employment in surgical operations and organ transplantations.

11. Use of a compound I as claimed in claim 1 for preparing a medicament for the preservation and storage of transplants for surgical measures.

12. Use of a compound I as claimed in claim 1 for preparing a medicament for the treatment of illnesses in which cell proliferation represents a primary or secondary cause.

13. A medicine, containing an effective amount of a compound I as claimed in claim 1.

## Revendications

1. Guanidides d'acides alkylcarboxyliques substitués par un groupe phényle portant des groupes perfluoroalkyle de formule I 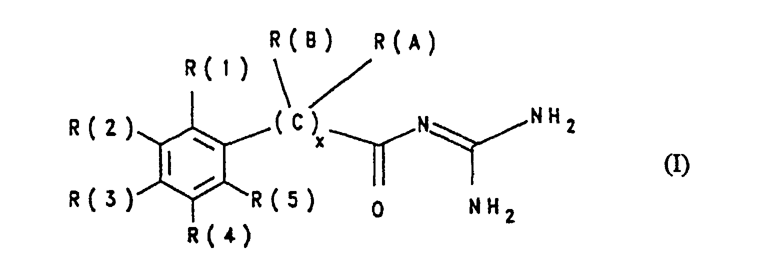 dans laquelle
R(A) représente un atome d'hydrogène, F, Cl, Br, I, CN, ou un gp OR(6), alkyle en C₁-C₄, -C_{b}F_{2b+1} ou NR(7)R(8),
b est 1, 2, 3 ou 4;
R(6) représente un atome d'hydrogène ou un groupe allyle en C₁-C₄, -C_{b}F_{2b+1}, phényle ou benzyle, les groupes aromatiques étant non substitués ou substitués par 1 à 3 substituants choisis dans le groupe constitué par F, Cl, CF₃, méthyle, méthoxy et NR(9)R(10);
R(9) et R(10) représentent H, CH₃ ou CF₃;
R(7) et R(8) ont indépendamment l'un de l'autre la signification de R(6),
R(B) a indépendamment la définition de R(A),
x est égal à 2,
R(1) représente un atome d'hydrogène ou un groupe allyle en C₁-C₄, -(O)ₜ-C_{b}F_{2b+1}, F ou Cl;
b est égal à 1, 2, 3 ou 4;
t est égal à 0 ou 1;
R(2), R(3), R(4), R(5) ont indépendamment les uns des autres la définition de R(1), à condition cependant qu'au moins l'un des substituants R(1), R(2), R(3), R(4) et R(5) soit un groupe -C_{b}F_{2b+1},
et leurs sels pharmaceutiquement acceptables.

2. Procédé de préparation d'un composé I selon la revendication 1, **caractérisé en ce que** l'on fait réagir un composé de formule II 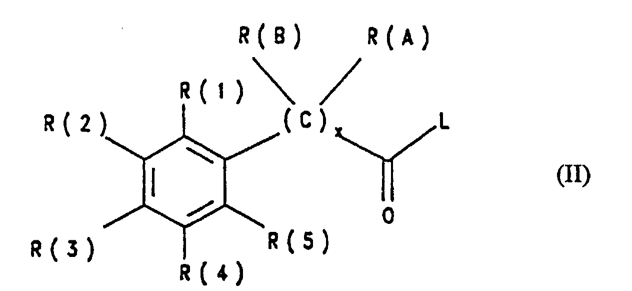 dans laquelle R(1) à R(5) et R(A) et R(B) ont la signification donnée dans la revendication 1 et L représente un groupe partant facile à substituer par substitution nucléophile,
avec de la guanidine,
et **en ce que** l'on transforme éventuellement le produit en un sel pharmaceutiquement acceptable.

3. Utilisation d'un composé I selon la revendication 1 pour la préparation d'un médicament destiné au traitement d'arythmies.

4. Utilisation d'un composé I selon la revendication 1 pour la préparation d'un médicament destiné au traitement ou à la prophylaxie de l'infarctus du myocarde.

5. Utilisation d'un composé I selon la revendication 1 pour la préparation d'un médicament destiné au traitement ou à la prophylaxie de l'angine de poitrine.

6. Utilisation d'un composé I selon la revendication 1 pour la préparation d'un médicament destiné au traitement ou à la prophylaxie d'états ischémiques du coeur.

7. Utilisation d'un composé I selon la revendication 1 pour la préparation d'un médicament destiné au traitement ou à la prophylaxie d'états ischémiques du système nerveux périphérique et central et de l'attaque d'apoplexie.

8. Utilisation d'un composé I selon la revendication 1 pour la préparation d'un médicament destiné au traitement ou à la prophylaxie d'états ischémiques d'organes périphériques et des membres.

9. Utilisation d'un composé I selon la revendication 1 pour la préparation d'un médicament destiné au traitement d'états de choc.

10. Utilisation d'un composé I selon la revendication 1 pour la préparation d'un médicament à utiliser au cours d'opérations chirurgicales et de greffes d'organes.

11. Utilisation d'un composé I selon la revendication 1 pour la préparation d'un médicament pour la conservation et le stockage de greffons destinés à des mesures chirurgicales.

12. Utilisation d'un composé I selon la revendication 1 pour la préparation d'un médicament destiné au traitement de maladies dans lesquelles la prolifération de cellules représente une cause primaire ou secondaire.

13. Médicament contenant une quantité active d'un composé I selon la revendication 1.
